Europäisches Patentamt

European Patent Office

Office européen des brevets

(19)

(11) Publication number: **0 025 649**
**B1**

## EUROPEAN PATENT SPECIFICATION

(12)

(45) Date of publication of patent specification: **26.10.83**

(21) Application number: **80302896.8**

(22) Date of filing: **21.08.80**

(51) Int. Cl.³: **A 61 K 35/78** //(A61K35/78, 33/30)

(54) **Pharmaceutical composition containing Sanguinaria and Galangal suitable for treatment of periodentitis and tumours.**

(30) Priority: **23.08.79 GB 7929364**

(43) Date of publication of application:
**25.03.81 Bulletin 81/12**

(45) Publication of the grant of the patent:
**26.10.83 Bulletin 83/43**

(84) Designated Contracting States:
**AT BE CH DE FR GB IT LI LU NL SE**

(56) References cited:
**BE - A - 874 607**
**US - A - 209 331**
**US - A - 2 334 830**

(73) Proprietor: **Orewa Inc.**
**Suite 707 Palacio Hotel**
**Estoril (PT)**

(72) Inventor: **Collins, Keith Reginald**
**Suite 707 Palacio Hotel**
**Estoril (PT)**

(74) Representative: **Dixon, Donald Cossar et al,**
**Gee & Co. Chancery House Chancery Lane**
**London WC2A 1QU (GB)**

Courier Press, Leamington Spa, England.

## Pharmaceutical compositions containing sanguinaria and galangal suitable for treatment of periodontitis and tumours

This invention relates to pharmaceutical compositions which are particularly effective in the treatment of periodontal and dental diseases malignant and benign skin tumours, and other conditions including malignant tumors of the breast, vulva and penis, bilharziasis, and skin infections by pseudomonas aeruginosa, cocci and fungi.

Periodontal disease is a major problem in modern dental practice. In fact, it can be said that practically all dental patients suffer from some type of periodontal disease—and that is the main cause for the loss of teeth in adults.

The only form of treatment to deal with periodontitis, to date, is surgery and that provides an undesirably high recurrence rate.

Most known treatments for skin cancer involve surgery to excise the tumours or radiotherapy to destroy affected cells. However, these treatments are not only psychologically unattractive to patients but, also, surgery involves anesthesia and usually leaves unsightly scarring, whereas radiotherapy is usually debilitating, involves the destruction of healthy tissue in most cases, and frequently leaves surface scarring. Surface scarring is particularly undesirable in cases effecting the patient's face and, unfortunately, skin cancer usually occurs in those areas, such as the face, which are most exposed to sunlight. Various substances such as nitrogen-mustards and pterins have been proposed for chemotherapy treatment of tumours, but no satisfactory results have been produced, at least partly because healthy cells may also be affected.

U.S. Patent 209,331 (1878) disclosed the use of equal portions in bulk of zinc chloride, bloodroot and kerosene oil for the treatment of cancers and sores. U.S. Patent No. 2,344,830 (1944) discloses a composition consisting of (a) zinc chloride, (b) stibnite (antimony trisulfide), (c) *Sanguinaria Canadensis* (presumably an extract thereof, but this is not made clear, nor from which part of the plant) and (d) water, for fixing diseased tissue prior to surgical excision. Belgian 874,607 discloses a composition consisting of (a) 0.1 to 40 weight % of a specified extract of *S. Canadensis*, (b) 0.1 to 30 weight % of zinc chloride, and (c) a medium such as a toothpaste base; this is beneficial to the mouth cavity.

The use of an extract of *S. Canadensis* together with zinc chloride is thus known to be beneficial for applying to various tissues, but not specifically for treating gingivitus.

We have found that a superior composition for this and other curative purposes is produced by incorporation of the plant extract galangal.

According to the invention a pharmaceutical composition comprises zinc chloride, the active ingredients of sanguinaria and galangal, and an excipient.

The excipient is preferably purified water.

It is preferred to include an excipient such as petroleum jelly in such a proportion as to produce an ointment-like consistency. A minor quantity, e.g. 2% by weight of sodium carboxymethyl cellulose may be incorporated.

Optimum results can be obtained from a composition having the following constituents, and hereinafter referred to as "Composition KC101":

| | |
|---|---|
| Zinc chloride | 45% |
| Deionized or demineralized water | 37% |
| Sanguinaria (dried rootstock of *Sanguinaria Canadensis*) | 10.8% |
| Galangal (dried rootstock of *Alpinia Officinarium*) | 7.2% |

The invention also provides a method of preparing the pharmaceutical composition.

Method of manufacture of composition KC101 Utmost care should be exercised in the handling, weighing and mixing of the components. Adequate ventilation and/or dust control (vacuum) may be necessary.

Zinc chloride is dissolved in the deionized water in a borosilicate heat-resistant glass container. The solution is filtered through a coarse sintered glass filter, collecting the filtered solution in a borosilicate glass container. Sanguinaria and galangal which have been passed through a 100 mesh bolting cloth are weighed out. This is done as follows:

a) an initial weighing of slight excess,
b) screening to 100 mesh,
c) reweighing to provide the amount required in the formula.

In operation, an excess is removed from the bulk container adequate to provide sufficient screening material for the product. The whole amount removed from the bulk containers should be recorded on a materials control card.

The excess, after screening, is discarded and not returned to the bulk container.

Mixing:

The sanguinaria and the galangal are mixed either in a plastic bag of a stainless steel container. The resulting mixed powder is placed in the pan of a laboratory mixer.

The zinc chloride solution is added to the mixed powder in increments of approximately 300ml. Thorough mixing is carried out following each addition, until the powder has absorbed the liquid. Thorough mixing is, again, carried out when all of the zinc chloride solution has been added to the powder.

Composition KC101 is a dark brown paste with a pleasant odour. This paste remains unaltered without any preserving agents, and needs no refrigeration. Its consistency is not affected by changes either in temperature or in relative humidity.

Ointments can be prepared using Composition KC101 and petroleum jelly or any other adequate excipient.

Composition KC101 is disinfectant, antiseptic, caustic and escharotic in its action, depending on its concentration.

It is believed that the effective properties of Composition KC101 are derived primarily from the combined active constituents of zinc chloride, sanguinaria and galangal.

Specific treatments, employing the pharmaceutical composition, will now be described:

1. Treatment of periodontal diseases

a) A periodontal disease such as gingivitis or one of the various stages of periodontitis is diagnosed during inspection and thorough cleaning of the mouth and, particularly, the gingiva and teeth.

b) The area to be treated is dried and isolated, and a layer of not less than 1mm thick of Composition KC101 is applied to the diseased area, and left in position for at least 10 minutes, after which period the paste is rinsed off and the patient's mouth is cleaned by suction.

c) This process is repeated, usually one to four times, at periods of 2 to 7 days, at the dental surgeon's discretion, until the disease appears to be clinically cured.

d) Subsequent visual checks are made to confirm the cure.

Notes:

1. As an alternative—regarding the time of treatment—in b), a dental dressing may be applied over Composition KC101, if a longer period of contact with the paste is felt to be required, for example, in a severe case of periodontitis.

2. In cases of severe periodontitis with deep pocket formations, another process of application may be used, in which small pieces of sterilized cotton strings are saturated with Composition KC101 and inserted in the periodontal pocket for 10 to 15 minutes. After that period, the piece of cotton string is removed, and the dental surgeon proceeds to the regular rinsing and cleaning of the mouth by suction.

3. According to the state of the disease and the individual response, the dental surgeon may use Composition KC101 full strength or diluted—in various percentages—in distilled water or glycerine.

4. Treatment with Composition KC101 has definite advantages over surgical treatment and, furthermore, the results obtained are equal to and, in most cases, superior to those obtained by surgery. The time for healing after treatment is also considerably shorter than that required by surgery.

2. Treatment of dental caries

a) After dental examination and detection of a caries, the carious cavity is thoroughly cleaned and disinfected. Composition KC101 is then applied to the cavity walls. A provisional occlusive dressing is applied over the carious cavity and left for a period of 7 to 15 days.

b) When the provisional dressing is removed, what remains of Composition KC101—a brown dust—is removed, and,

c) The dental surgeon proceeds to the porcelain filling of the cavity or to the dental restoration with amalgam.

Notes:

1. At the dental surgeon's discretion, another treatment may be carried out, before step c), if the caries is particularly advanced.

2. The main advantages of this treatment over conventional treatments are:

I Treatment with Composition KC101 consists of 2 to 4 very short sessions (10 minutes, each, on average) as opposed to several long sessions (40 minutes, each, on average).

II Drilling is, as a rule, not required.

III No anesthesia is necessary—this treatment is painless.

IV No psychological preparation of the patient is needed, as is the case when anesthesia and drilling are to be expected.

V Treatment is carried out as easily in children as in adults.

VI Even milk teeth can be submitted to this treatment.

3. Treatment of skin tumours of the following kinds

Malignant

Squamos-cell carcinoma, Basal-cell carcinoma, Malignant melanoma, Sarcoma and Kaposi's sarcoma.

Benign

Kerato-acanthoma, Granuloma Pyogenicum, Pseudocarcinomatous hyperplasia and Common warts.

a) Clinical diagnosis of a tumour is confirmed by histological diagnosis (biopsy).

b) The lesion is measured, both superficially and subcutaneously.

c) A layer not less than 1mm thick of Composition KC101 is applied on and, to recognized safety margins at the surgeons discretion, around the lesion.

d) A non-occlusive dressing is applied for a period of about 24 hours, to encompass the period of initial activity of the paste, which is approximately 20 hours.

e) After the 24 hour period, the dressing is removed, and the lesion cleaned.

f) Fresh dressings are applied, every other day, for a week to encompass the further period of activity of the paste.

g) After that period there is a definite demarcation isolating the necrotic tumour from the healthy tissue. The tumour is, then, easily removed using only forceps.

h) The resulting ulceration is measured and inspected and, if satisfactory, is redressed and allowed to heal.

i) Shortly before or just after complete healing, a final biopsy is performed to confirm the clinical evidence of cure.

j) Subsequent regular checks are made over the conventional period of five years.

Notes:

1. Regarding g), the paste has a higher affinity for neoplastic tissue than for normal tissue. The tumour isolation results from the acute demarcating inflammation provoked by the paste (skin necrosis, following a traumatism or burn, requires at least 3 weeks for isolation and sloughing off). In some cases, the tumour will even slough off with the dressing as that dressing is being removed.

2. Regarding h), if the measurements of the resulting ulceration do not conform to the recognized safety margins, again at the surgeon's discretion, or if there is an apparent persistence of cancer cells, steps c) to g) are repeated.

3. In the case of massive tumours, several applications of the paste are made, at periods of say three or four days, according to the surgeon's diagnosis of each case, and progress is inspected accordingly.

4. The same technique is followed for treatment of malignant tumours of the breast, vulva and penis.

4. Treatment of Bilharziasis (Schistosomiasis)

This is a very grave infestation, widespread through Asia, Africa and Tropical America and may be a precancerous disease.

a) Capsules are prepared from a mixture of 100mg of Composition KC101 and gelatin crystals, as a buffer, in gelatin capsules.

b) A capsule is administered to the infected patient morning and night, after meals, each day for about one week to a month. The internal action of the paste kills the eggs of the infesting worms which rapidly die off.

5. Treatment of the Pseudomonas aeruginosa

This usually affects surgical wards in hospitals and, being highly contagious and infectious, spreads rapidly. Current cures may take two weeks or more, and surgical activity must usually be suspended if this infection is present.

a) An ointment is prepared, comprising between 1% and 5% of Composition KC101 and petroleum jelly (excipient).

b) A layer of ointment is applied to and around all areas, usually wounds, which are infected by Pseudomonas aeruginosa.

c) The treated areas are inspected, after about 24 hours and, in almost all cases, the infection should be terminated.

d) After a further 24 hours, a check is made to ensure that no further, or residual, infections by Pseudomonas aeruginosa exist; the infection can then be regarded as cleared.

Notes:

1. Regarding a), areas where infections by Pseudomonas aeruginosa might be expected may also be treated.

2. Regarding c), in the unlikely event of the infection remaining in a treated area, a further layer of more concentrated ointment is applied.

3. The ointments are used, in a similar manner, also for the treatment of infections by cocci and fungi.

In a development of the invention, initial tests have shown that the effectiveness of the composition may be improved by the incorporation of sodium carboxy-methyl cellulose in an amount of the order of 2% by weight. Particularly in the case of the ointment. This additive acts as a drying agent for the outer layer and has effectively stopped "running" after a period of about $1\frac{1}{2}$ hours. The additive, which may be incorporated during manufacture of the composition, has not presented any ecological problems and appears to have no chemical effect on the composition.

Further pharmaceutical uses for the composition according to the invention are being explored and, due to its suggested effectiveness as an anti-bacterial, anti-fungi, cytotoxic and immunopotentiator, further effective results are expected in the treatment of fungal diseases and microbial diseases, and use in general immunology should be promising.

Furthermore, work is in progress to synthesise the composition, which will, eventually, permit its research in general internal diseases.

Lastly, experiments have already shown that the composition is equally effective in the veterinary field generally, and specifically on horses and cattle.

**Claims**

1. A pharmaceutical composition comprising zinc chloride, the active ingredients of sanguinaria and galangal, and an excipient.

2. A composition according to Claim 1, in which said excipient is purified water.

3. A composition according to Claim 2, in which the constituents are in approximately the following proportions, by weight: zinc chloride 45%; sanguinaria 10.8%; galangal 7.2%; water 37%.

4. A composition according to any preceding claim, and further comprising an excipient such as petroleum jelly in such a proportion as to produce an ointment-like consistency.

5. A composition according to any of Claims 1 to 4, and further comprising a minor quantity of sodium carboxy-methyl cellulose.

6. A composition according to Claim 5, in which said minor proportion is approximately 2% by weight of the composition.

7. A pharmaceutical capsule containing a composition according to any of Claims 1 to 3, and a buffer.

8. A capsule according to Claim 7, in which said buffer comprises gelatin crystals.

9. A method of preparing a pharmaceutical composition as claimed in claim 1, comprising mixing active ingredients of sanguinaria and galangal, zinc chloride and excipient.

10. A method of preparing a pharmaceutical composition, comprising dissolving zinc chloride in purified water, screening and mixing sanguinaria and galangal and adding the zinc chloride solution to the mixture of sanguinaria and galangal to produce a paste.

11. A method according to Claim 10, in which the constituents are in the following approximate final proportions by weight: zinc chloride 45%; sanguinaria 10.8%; galangal 7.2%; purified water 37%.

12. A method according to Claim 10 or Claim 11, and further comprising the step of mixing the paste with an excipient such as petroleum jelly to produce an ointment.

13. A method according to any of Claims 10 to 12, and further comprising the step of adding a minor proportion of sodium carboxy-methyl cellulose.

14. A method according to Claim 13, in which said minor proportion is approximately 2% by weight of the composition.

**Revendications**

1. Composition pharmaceutique comprenant du chlorure de zinc, les principes actifs de la sanguinaire et du galanga, et un excipient.

2. Composition selon la revendication 1, dans lequelle ledit excipient est de l'eau purifiée.

3. Composition selon la revendication 2, dans laquelle les constituants sont approximativement dans les proportions suivantes en poids: chlorure de zinc 45%, sanguinaire 10,8%; galanga 7,2%: eau 37%.

4. Composition selon l'une des revendications précédentes, comprenant en outre un excipient tel que la gelée de pétrole dans une proportion telle qu'on obtienne la consistance d'une pommade.

5. Composition selon l'une des revendications 1 à 4, comprenant en outre une petite quantité de carboxy-méthyl cellulose de sodium.

6. Composition selon la revendication 5, dans laquelle ladite petite proportion est approximativement 2% en poids de la composition.

7. Gellule pharmaceutique contenant une composition selon l'une des revendications 1 à 3, et un tampon.

8. Gellule selon la revendication 7, dans laquelle ledit tampon comprend des cristaux de gélatine.

9. Procédé de préparation d'une composition pharmaceutique selon la revendication 1, dans lequel on mélange les principes actifs de la sanguinaire et du galanga, du chlorure de zinc et un excipient.

10. Procédé de préparation d'une composition pharmaceutique, dans lequel on dissout du chlorure de zinc dans de l'eau purifiée, on tamise et on mélange de la sanguinaire et du galanga, et on ajoute la solution de chlorure de zinc au mélange de sanguinaire et de galanga pour produire une pâte.

11. Procédé selon la revendication 10, dans lequel les constituants sont dans les proportions pondérales finales approximatives suivantes: chlorure de zinc 45%; sanguinaire 10,8%; galanga 7,2%; eau purifiée 37%.

12. Procédé selon l'une des revendications 10 et 11, comprenant en outre l'étape consistant à mélanger la pàte avec un excipient tel que la gelée de pétrole pour produire une pommade.

13. Procédé selon l'une des revendications 10 à 12, comprenant en outre l'étape consistant à ajouter une petite quantité de carboxy-méthyl cellulose de sodium.

14. Procédé selon la revendication 13, dans lequel ladite petite proportion est approximativement de 2% en poids de la composition.

**Patentansprüche**

1. Pharmazeutische Zusammensetzung, umfassend Zinkchlorid, die worksamen Bestandteile von Sanguinaria und Galangal und einen Exzipienten.

2. Zusammensetzung nach Anspruch 1, dadurch gekennzeichnet, daß der Exzipient gereinigtes Wasser ist.

3. Zusammensetzung nach Anspruch 2, dadurch gekennzeichnet, daß die Bestandteile annähernd in den folgenden Gewichtsanteilen vorliegen: Zinkchlorid 45%; Sanguinaria 10,8%; Galangal 7,2%; Wasser 37%.

4. Zusammensetzung nach einem der vorhergehenden Ansprüche, dadurch gekennzeichnet, daß sie weiters einen Exzipienten wie Petroleumgelee in einer solchen Menge enthält, daß eine salbenartige Konsistenz erzielt wird.

5. Zusammensetzung nach einem der Ansprüche 1 bis 4, dadurch gekennzeichnet, daß sie weiters eine kleinere Menge Natriumcarboxymethylcellulose enthält.

6. Zusammensetzung nach Anspruch 5, dadurch gekennzeichnet, daß die genannte kleinere Menge annähernd 2 Gew.-% der Zusammensetzung beträgt.

7. Pharmazeutische Kapsel, enthaltend eine Zusammensetzung nach einem der Ansprüche 1 bis 3 und einen Puffer.

8. Kapsel nach Anspruch 7, dadurch gekennzeichnet, daß der Puffer aus Gelatinekristallen besteht.

9. Verfahren zur Herstellung einer pharmazeutischen Zusammensetzung gemäß Anspruch 1, umfassend das Vermischen der wirksamen Bestandteile von Sanguinaria und Galangal, Zinkchlorid und Exzipient.

10. Verfahren zur Herstellung einer pharmazeutischen Zusammensetzung, dadurch gekennzeichnet, daß man Zinkchloride in gereinigtem Wasser löst, Sanguinaria und Galangal seibt und vermischt und dem Gemisch von Sanguinaria und Galangal die Zinkchloridlösung unter Bildung einer Paste zusetzt.

11. Verfahren nach Anspruch 10, dadurch gekennzeichnet, daß die Bestandteile in den folgenden annähernden End-Gewichtsanteilen vorliegen: Zinkchlorid 45%; Sanguinaria 10,8%; Galangal 7,2%; Wasser 37%.

12. Verfahren nach Anspruch 10 oder 11, dadurch gekennzeichnet, daß die Paste noch mit einem Exzipienten wie Petroleumgelee vermischt wird, um eine Salbe zu erzeugen.

13. Verfahren nach einem der Ansprüche 10 bis 12, dadurch gekennzeichnet, daß noch eine kleinere Menge Natrium-carboxymethylcellulose zugesetzt wird.

14. Verfahren nach Anspruch 13, dadurch gekennzeichnet, daß die gennante kleinere Menge annähernd 2 Gew.-% der Zusammensetzung beträgt.